# EUROPEAN PATENT APPLICATION

(11) **EP 4 734 121 A1**
(43) Date of publication of application: **29.04.2026**
(21) Application number: 24383180.7
(22) Date of filing: 28.10.2024
(51) Int. Cl.: G16H 20/10, G16H 50/20, G16H 50/30, G16H 10/60

(54) **CLINICAL DECISION SUPPORT SYSTEM FOR LIPIDIC CONTROL**

(71) Applicant: Instituto de Investigación Biomédica de Salamanca de la Fundación Instituto Ciencias de la Salud de Castilla y León, 37007 Salamanca (ES); Universidad de Salamanca, 37008 Salamanca (ES); Consorcio Centro de Investigación Biomédica en Red, 28029 Madrid (ES)
(72) Inventor: SANCHEZ FERNANDEZ, Pedro Luis, 37007 Salamanca (ES); OTERINO MANZANAS, Armando, 37007 Salamanca (ES); RINCON DIAZ, Luis Miguel, 37007 Salamanca (ES); SANCHEZ PUENTE, Antonio, 37007 Salamanca (ES); PEREZ SANCHEZ, Pablo, 37007 Salamanca (ES); ISIDORO GARCIA, María, 37007 Salamanca (ES); CEMBRERO FUCIÑOS, David, 37007 Salamanca (ES)
(74) Representative: Hoffmann Eitle

(57) **Abstract**

The present invention relates to a clinical decision support system comprising a processing unit in communication with a memory. The processing unit is configured to receive and process clinical data associated with a patient, which comprises medical records, biometric or physiological data, and/or currently prescribed medication information. The system computes personalized target lipid profile ranges based on the clinical data and medical guidelines stored in the memory. Preferably, the system receives pharmacogenetic analysis data and measured lipid profile values from patient blood tests. The processing unit compares the measured lipid values with the personalized target ranges and, if deviations are found, computes a list of potential medication treatments. This list is sorted based on the estimated effects of each treatment on the lipid profile values, the different between measured and objective lipid profile values, on the clinical data and/or on the pharmacogenetic analysis. The system further outputs data related to the personalized lipid ranges, the comparison of measured values, and/or the suggested medication treatments.

## Description

### Technical field of the invention

The present invention relates to the field of medical technology and diagnostics, specifically to clinical decision support systems implemented in devices and/or computer programs. More particularly, it concerns a clinical decision support system for lipid control that computes personalized target lipid profile values and provides medication recommendations.

### Background of the invention

Cardiovascular disease (CVD) remains the leading cause of mortality globally, comprising a wide range of conditions originating in the heart and blood vessels, wherein some of these conditions are coronary atherosclerosis, cerebrovascular disease, and peripheral arterial disease. Atherosclerosis is the fundamental pathophysiological process behind many cardiovascular conditions, involving multiple factors such as genetic predisposition, systemic inflammation, and notably, elevated levels of low-density lipoprotein cholesterol (LDL-C).

Cholesterol esters, insoluble in aqueous media, are transported in the bloodstream by lipoproteins, which are fundamentally divided by their density and their infiltrative capacity in the arterial intima. Cholesterol bound to low density lipoproteins (LDL-C) by its presence in quantitative terms and its size plays a fundamental role in atherosclerosis as an etiological factor of atherosclerosis. The loss of glycocalyx in the arterial wall facilitates the binding of apoprotein B, which confers a structural function to LDL particles and the passage of these particles into the arterial intima. There, they undergo oxidative modifications, transforming into oxidised LDL (oxLDL) rich in oxidised phospholipids, triggering an inflammatory response, activating macrophages which ingest the oxLDL to become foam cells, producing the first stages of atherosclerosis. The arrival of muscle cells and their dedifferentiation allows the formation of a fibrous cap that separates the lipid core from the bloodstream in atherosclerotic plaques. If this sheath ruptures, the plaque becomes unstable and the lipid core is exposed to the bloodstream, which activates the coagulation cascade leading to thrombosis and acute clinical events such as myocardial infarction or stroke.

Epidemiological data underscore the significant impact of CVD on public health and economies, since despite advances in medical therapies and preventive strategies, CVD continues to account for a substantial percentage of deaths annually. For instance, in several countries, it surpasses other leading causes of death, including cancer and respiratory diseases. The economic burden is equally profound, with costs arising from healthcare expenditures, loss of productivity, and long-term care needs.

Current clinical guidelines advocate the reduction of LDL-C levels as a primary strategy in both the prevention and treatment of CVD. Meta-analyses of clinical trials demonstrate that LDL-C reduction correlates with a reduced risk of cardiovascular events; specifically, a 40 mg/dL reduction in LDL-C levels can lead to a 22% reduction in cardiovascular events and a 10% reduction in cardiovascular mortality. Importantly, no lower threshold has been identified below which LDL-C reduction ceases to produce prognostic benefits without adverse effects related to plasma cholesterol depletion.

Despite the availability of various lipid-lowering agents, including statins, ezetimibe, and PCSK9 inhibitors, a significant proportion of patients do not achieve recommended LDL-C targets, and studies reveal that nearly 50% of patients in secondary prevention fail to reach guideline-directed LDL-C levels. Factors contributing to this shortfall include inadequate dosing, poor adherence to therapy, and lack of personalized treatment approaches.

One major limitation in current lipid management is the reliance on generalized treatment protocols that may not account for individual patient variability. In fact, the assumptions we make on the reduction of lipid-lowering medications when used in combination are based on estimates based on pivotal clinical trials, with sometimes clinically significant discrepancies from what is observed in the real-world evidences studies. Standardized targets and therapies often overlook unique clinical factors such as comorbid conditions, concomitant medications, and individual responses to drugs. This can lead to suboptimal lipid control, increased risk of adverse effects, and ultimately, insufficient prevention of cardiovascular events.

Moreover, current clinical decision support systems are not prepared to identify potential drug-drug interactions and contraindications, which is particularly critical given the prevalence of polypharmacy in patients with multiple health conditions.

Genetic variations, on the other hand, can markedly influence drug metabolism, efficacy, and the risk of adverse reactions. Therefore, clinicians may prescribe medications that are less effective or have a higher likelihood of causing side effects in certain individuals. This not only impacts patient outcomes but can also reduce adherence to treatment due to intolerable adverse effects.

Additionally, the monitoring of lipid profiles and adjustment of therapy are often not as dynamic or responsive as needed. Delays in recognizing that a patient is not achieving target lipid levels can postpone necessary adjustments to their treatment plan.

There is, therefore, an emerging need for improved clinical decision support systems in lipid management that properly addresses and overcomes these challenges. Addressing these shortcomings is essential to enhance the efficacy of lipid-lowering strategies, reduce the incidence of cardiovascular events, and improve overall patient outcomes.

### Summary of the invention

The present invention relates to a clinical decision support system (CDS) designed to overcome the limitations of current approaches in in the treatment and monitoring of patients with dyslipidaemia, such as generic lipid-lowering therapies, inadequate patient adherence, and the absence of individualized treatment strategies.

Current lipid management practices often rely on standard protocols that fail to account for individual patient variability, such as comorbid conditions, medication interactions, and genetic predispositions. These generalized approaches can lead to suboptimal lipid control, increased risk of adverse effects, and insufficient reduction of cardiovascular events. The system of the invention addresses these issues by allowing to receive and process detailed patient-specific clinical data, which preferably comprises medical history, biometric data, physiological measurements, and/or prescribed medications.

Additionally, in some embodiments of the invention, the CDS system integrates pharmacogenetic analysis, which allows the system to adjust lipid-lowering medications based on individual genetic determinants. By incorporating genetic information, the system minimizes the risk of adverse drug reactions and optimizes the efficacy of lipid-lowering therapies, solving the current problem of prescribing medications without considering patient-specific genetic factors. This personalized approach reduces the likelihood of side effects, improving medication adherence and patient outcomes.

Furthermore, the system of the invention is adequate for monitoring the patient's lipid profile through regular blood analyses, and, preferably, by comparing measured lipid levels with personalized target ranges derived from clinical guidelines stored in the memory, such as, but not limited to, the European Society of Cardiology (ESC) and/or the European Atherosclerosis Society (EAS) Guidelines. When lipid levels deviate from the target ranges, the system can generate a ranked list of potential medication treatments, preferably taking into account one or more of the following parameters: the estimated effect of each medication treatment on the lipid profile values, the pharmacogenetic data, clinical history, and the degree of deviation from the target lipid levels.

In some embodiments of the invention, the system further solves the issue of poor adherence to medication by analysing patient data related to adherence patterns. Preferably, if deviations from target lipid levels are detected, the system can identify whether they are linked to non-compliance with prescribed medications.

This invention provides a comprehensive and personalized approach to lipid management by integrating clinical data, pharmacogenetic analysis, and dynamic monitoring of lipid profiles in a clinical decision support system for lipid and/or cholesterol control. It addresses the shortcomings of current systems, such as generalized treatment protocols, lack of personalized therapy, and delayed adjustments to lipid-lowering treatments, offering a more precise and effective tool for reducing cardiovascular risks in patients with dyslipidaemia.

In a first aspect of the invention, a clinical decision support system comprising a processing unit in communication with a memory is disclosed. The processing unit is configured to receive clinical data associated with a patient, which includes medical records, physiological data, and/or data related to medication currently prescribed to the patient. The processing unit is further configured to compute personalized ranges of target lipid profile values by taking the patient's clinical data as input and computing the personalized target lipid profile values according to medical guidelines. Optionally, it is configured to receive a pharmacogenetic analysis of the patient. It is further configured to receive measured lipid profile values from a blood analysis. The processing unit is also configured to compare the measured lipid profile values with the personalized target lipid profile values. If one or more measured lipid profile values lie outside the corresponding personalized ranges, it computes a list of medication treatments, wherein the list is sorted based on the estimated effect of each medication treatment on the lipid profile values, the difference between the measured lipid profile values and the personalized target lipid profile values, at least part of the clinical data, and preferably also on the pharmacogenetic analysis. Furthermore, the processing unit is configured to output data related to the personalized target lipid profile values, the comparison or difference between the measured lipid profile values and the personalized ranges of target lipid profile values, and/or the sorted list of medication treatments.

According to one or more embodiments of the invention, the lipid profile values comprise LDL cholesterol, total cholesterol (TC), triglycerides, HDL cholesterol, VLDL cholesterol, remnant cholesterol particles, the ratio TC/HDL, the ratio LDUHDL, lipoprotein (a), apolipoprotein A1, apolipoprotein B, and/or non-HDL cholesterol levels, preferably comprising at least LDL cholesterol.

According to one or more embodiments of the invention, the pharmacogenetic analysis comprises data of the tolerance of the patient towards dyslipidaemia medication and/or data related to the genetic predisposition and individualized response of the patient towards a particular lipid profile.

According to one or more embodiments of the invention, the clinical data comprises data related to the adherence of the patient to the prescribed medication, and the processing unit of the system is further configured to output data related to the adherence of the patient to the prescribed medication if the measured lipid profile of the patient diverges from the personalized target lipid profile.

According to one or more embodiments of the invention, the clinical data comprises risk factors, and the computation of the personalized target lipid profile further takes said risk factors as input.

According to one or more embodiments of the invention, a risk factor is the prevalence or not of diabetes in the patient and the type of diabetes.

According to one or more embodiments of the invention, a risk factor is the existence of a medical record related to one or more previous atherothrombotic events, coronary heart diseases, peripheral arterial diseases, and/or cerebral ischaemia.

In some embodiments of the invention, the clinical decision support system may receive more than one measured lipid profile from blood analytics of the patient, along with a time reference or date of said blood analytics. Instead of comparing the lipid profile values of the patient with the personalized target lipid profile, the processing unit is configured to compare the last measured lipid profile values of the patient with the personalized ranges of target lipid profile values, and if the last measured values lie outside the personalized ranges, it provides a list of medication treatments. This list is sorted based on the evolution of the measured lipid profiles over time, the effect of each medication treatment on the patient's lipid profile, the difference between the measured lipid profile values and the personalized ranges of target lipid profile values, at least part of the clinical data, and, preferably also the pharmacogenetic analysis.

According to one or more embodiments of the invention, the physiological data comprises data related to the patient's sex and/or age.

According to one or more embodiments of the invention, the prescribed medication comprises dyslipidaemia medication, hypolipidemic medication, and/or anti-hypolipidemic medication.

According to one or more embodiments of the invention, at least medical records and the medication currently prescribed to the patient are received as clinical data. If the medical records indicate certain current medical conditions and the prescribed medication includes lipid-lowering medication, the processing unit is further configured to output a safety warning indicating possible secondary effects of the medication, wherein the medical conditions preferably comprise at least one of myalgia, rhabdomyolysis, and/or high transaminases.

According to one or more embodiments of the invention, the personalized ranges of target lipid profile comprise at least one first threshold subrange. If at least one value of the measured lipid profile of the patient falls within this threshold subrange, and the patient is not currently prescribed any dyslipidaemia medication, the system does not provide a list of medication treatments and instead outputs a recommendation for a healthy lifestyle.

According to one or more embodiments of the invention, the personalized ranges of target lipid profile comprise at least one second threshold subrange. If at least one value of the measured lipid profile of the patient falls within this threshold subrange, and the patient is currently prescribed one or more dyslipidaemia medications, the system outputs a recommendation for a healthy lifestyle and provides a list of medication treatments. The list is preferably sorted based on the effect of each medication treatment on the patient's lipid profile, the threshold subrange in which the measured lipid profile lies, part of the clinical data, and/or the pharmacogenetic analysis.

According to one or more embodiments of the invention, a computer program comprises instructions which, when executed by a processor, cause the processor to carry out the steps included in the configuration of the processing unit according to any one of the previous claims.

According to one or more embodiments of the invention, a computer-readable storage medium comprises instructions which, when executed by a processor, cause the computer to carry out the steps included in the configuration of the processing unit according to any one of the previous claims.

### Brief description of the drawings

To enable a better understanding of the present disclosure, and to show how the present disclosure may be carried out, reference will now be made, by way of example only, to the accompanying schematic drawings, wherein:
Figure 1 shows a schematic diagram of the medical process wherein a clinical decision support (CDS) system according to one or more embodiments of the invention may be integrated into.
Figure 2 shows a flow chart summarizing the steps performed since a patient performs a lipid analytics until receives an optimized treatment, by using a CDS system according to one or more embodiments of the invention.
Figure 3 shows a schematic diagram of the logic steps followed by a CDS system according to one or more embodiments of the invention.
Figure 4 shows a table with different lipidic medication and their concentration for different intensity levels, as well as medication for inhibiting PCSK9.
Figure 5 displays a table with exemplary cholesterol analytics results.
Figure 6 displays a bar chart displaying theoretic LDLc reduction, in percentage, for different medication types, wherein the vertical doted line represents the LDLc value objective
Figure 7 displays another bar chart displaying theoretic LDLc reduction, in percentage, for different medication types, wherein the vertical doted line represents the LDLc value objective

### Description of the invention

### Definitions

As used herein, "a" or "an" means "at least one" or "one or more."

It is noted that the term "about", if used herein, refers to +/- 30%, preferably +/- 20%, preferably +/- 15%, more preferably +/- 10%, of the indicated referred value. It may also refer to the exact value, that is, +/- 0% or +/- the standard measurement error. Also, any quantity, composition percentages and/or dimension mentioned herein is understood to present an error of +/- 30%, preferably +/- 20%, preferably +/- 15%, more preferably +/- 10%, more preferably +/- 5% or +/-0% of the indicated value. It may also refer to the exact value, that is, +/- 0% or +/- the standard measurement error.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together.

When used herein the term "comprising" can be substituted with the term "containing" or "including" or sometimes when used herein with the term "having".

When used herein "consisting of' excludes any element, step, or ingredient not specified in the claim element. When used herein, "consisting essentially of" does not exclude materials or steps that do not materially affect the basic and novel characteristics of the claim.

Unless defined otherwise, all technical and scientific terms used herein have the same meaning as is commonly understood by one of ordinary skill in the art to which this invention belongs. All patents, applications, published applications and other publications referred to herein are incorporated by reference in their entirety. If a definition set forth in this section is contrary to or otherwise inconsistent with a definition set forth in applications, published applications and other publications that are herein incorporated by reference, the definition set forth in this section prevails over the definition that is incorporated herein by reference.

The term "clinical decision support system," or CDS system, refers to any software application, hardware device, or integrated platform designed to assist healthcare professionals in making informed clinical decisions by processing and analysing patient-specific data. In the context of this invention, the CDS system is specifically aimed at managing and optimizing lipid control in patients, preferably focusing on the monitoring of lipid profiles, adjusting treatments, and providing personalized medication recommendations. This may comprise, but is not limited to, systems that deliver personalized assessments, diagnostic support, alerts, or treatment plans based on clinical guidelines, patient lipid levels, pharmacogenetic information, and other relevant clinical data.

The term "clinical data", in the context of the invention, refers to any information related to a patient's health status, medical history, or healthcare interventions that can be utilized for clinical assessment and decision-making. This may comprise, but is not limited to, medical records, laboratory test results, imaging studies, biometric measurements (such as blood pressure, heart rate), physiological data, and details of medications currently prescribed to the patient. Some examples comprise electronic health records, blood analytics, urine analytics data, patient questionnaires, vital signs from wearable devices, and/or data from remote monitoring systems.

The term "lipid profile", in the context of the invention, refers to one or more blood test results that measure the levels of specific lipids in the bloodstream to assess cardiovascular health and risk. This may comprise, but is not limited to, measurements of total cholesterol (TC), low-density lipoprotein cholesterol (LDL cholesterol, or LDLc), high-density lipoprotein cholesterol (HDL cholesterol, or HDLc), triglycerides, very low-density lipoprotein cholesterol (VLDL cholesterol), remnant cholesterol particles, the ratio of total cholesterol to HDL cholesterol (TC/HDL), lipoprotein(a), apolipoprotein A1, apolipoprotein B, and/or non-HDL cholesterol levels.

The term "pharmacogenetic analysis", in the context of the invention, refers to the examination of an individual's genetic information to predict their response to specific medications, aiming to optimize drug efficacy and minimize adverse effects. This may comprise, but is not limited to, genetic tests that identify variants affecting drug metabolism, transport, and/or target receptors relevant to lipid-lowering therapies. Examples may include analysis of genes such as SLCOL B1 for statin-induced myopathy risk, CYP enzymes influencing drug metabolism, or genetic markers associated with lipid disorders and treatment responses.

The term "lipid-lowering medication", in the context of the invention, refers to any pharmaceutical agent prescribed to reduce lipid levels in the blood, particularly cholesterol and triglycerides, to manage or prevent cardiovascular diseases. This may comprise, but is not limited to, statins, fibrates, niacin, bile acid sequestrants, cholesterol absorption inhibitors like ezetimibe, omega-3 fatty acid supplements, PCSK9 inhibitors, and/or other agents that may modify lipid metabolism. Examples include commonly used medications such as atorvastatin, rosuvastatin, gemfibrozil, and evolocumab.

The term "adherence of the patient to the prescribed medication", in the context of the invention, refers to the extent to which a patient correctly follows medical advice regarding timing, duration, dosage, and frequency of medication intake.

The term "risk factors", in the context of the invention, refers to any attributes, characteristics, or exposures that increase an individual's likelihood of developing a disease or health disorder, preferably cardiovascular conditions, more preferably lipid or cholesterol related pathologies. This may comprise, but is not limited to, medical conditions, genetic predispositions, lifestyle habits, physiological factors and/or demographic factors. Examples of risk factors may include the presence of diabetes (type 1 or type 2), hypertension, pregnancy, smoking, obesity, family history of cardiovascular disease, advanced age, male sex, physical inactivity, and elevated lipid levels.

### Description

Cardiovascular disease (CVD) remains the leading cause of mortality worldwide, driven by conditions such as coronary atherosclerosis, cerebrovascular disease, and peripheral arterial disease. A key factor in the development of atherosclerosis is elevated low-density lipoprotein cholesterol (LDL-C), which, through oxidative modifications, promotes plaque formation in arterial walls. Despite advances in treatment, such as the use of lipid-lowering agents like statins and PCSK9 inhibitors, a significant proportion of patients fail to reach recommended LDL-C targets. Poor adherence to therapy, inadequate dosing, and the lack of personalized treatment approaches contribute to this shortfall. The economic burden of CVD is substantial, as the disease not only leads to significant healthcare costs but also reduces productivity and increases long-term care needs. Therefore, there is an urgent need for advanced clinical decision support system (CDS) systems that can offer personalized lipid management, address patient adherence, and provide timely adjustments to treatment plans to better prevent cardiovascular events.

The present invention addresses the aforementioned challenges by providing a CDS system aimed at improving the treatment and monitoring of patients with dyslipidaemia by addressing the shortcomings of current lipid management practices. This system allows for the integration of patient-specific clinical data, that may comprise, but is not limited to, medical history, biometric measurements, and/or, in some embodiments, pharmacogenetic analysis, to offer personalized treatment recommendations. The CDS system can dynamically monitor the patient's lipid profile, comparing measured lipid levels with personalized target ranges based, preferably, on clinical guidelines. When deviations are detected, the system is configured to generate a ranked list of medication treatments tailored to the patient's specific clinical and genetic profile. Additionally, in some embodiments the system can identify potential issues related to medication adherence, further optimizing treatment. By incorporating these innovative features, the invention provides a more effective and individualized approach to lipid control, ultimately improving cardiovascular outcomes for patients with dyslipidaemia.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

Therefore, in a first aspect of the invention, a clinical decision support (CDS) system comprising a processing unit in communication with a memory is disclosed. The processing unit of the CDS system is configured to receive clinical data associated to a patient, wherein said clinical data comprises medical records, biometric data, physiological data, and/or data related to medication currently prescribed to the patient.

The term "clinical data" is intended to cover a broad scope of patient-specific information, which may comprise, but is not limited to, detailed medical history, laboratory results, imaging data, diagnostic reports, and/or other relevant clinical records. The biometric or physiological data may encompass metrics such as, but not limited to, heart rate, blood pressure, weight, body mass index (BMI), cholesterol levels, and other markers of cardiovascular health.

Data related to medications currently prescribed to the patient may include various forms of drug-related information, such as the type of medication, dosage, administration frequency, drug-drug interactions, and potential side effects. This data may be derived from electronic health records (EHRs) or entered manually into the system by healthcare professionals.

It is further noted that the system is designed to handle a range of data types, allowing it to accommodate different formats and sources, ensuring the system's interoperability with existing medical databases and health information systems. The clinical data may be received via manual input, automated data integration with healthcare information systems, or other means of data transmission such as cloud-based platforms or external databases.

In some embodiments, the system may incorporate real-time data acquisition from wearable devices or remote monitoring systems, enhancing the breadth and timeliness of the clinical data received by the processing unit. This would enable continuous monitoring of physiological parameters and medication adherence, providing a more dynamic and comprehensive patient profile.

The processing unit of the CDS system of the invention is further configured to compute personalized ranges of target lipid profile values taking as input the clinical data of the patient and wherein said personalized target lipid profile values are preferably computed according to medical guidelines values stored in the memory. Alternatively, the lipid profile may be received, or manually or automatically provided to the system.

The personalized ranges of lipid profile values may include, but are not limited to, target values for low-density lipoprotein cholesterol (LDL-C), high-density lipoprotein cholesterol (HDL-C), the ratio LDL-C/HDL-C, total cholesterol, triglycerides, and other relevant lipid biomarkers.

The computation of these personalized target lipid profile values are preferably performed by taking into account medical guidelines, more preferably stored in the memory or in a server. These guidelines may comprise standardized recommendations from clinical organizations, such as, but not limited to, the European Society of Cardiology (ESC), the European Atherosclerosis Society (EAS), the American Heart Association (AHA), or other relevant medical bodies. These guidelines may define general target lipid ranges for different patient populations, considering factors such as age, gender, medical history, and risk of cardiovascular events, among other options.

It is further noted that the system dynamically adapts these generalized guideline values to the individual patient's profile by integrating the clinical data received. The processing unit may account for specific variables such as genetic predisposition, comorbidities, lifestyle factors, and any existing or prior treatments. These personalized adjustments ensure that the computed lipid profile targets are tailored to the patient's unique condition and therapeutic needs.

In some embodiments, the processing unit may employ machine learning algorithms or predictive models to refine the accuracy of the computed personalized lipid targets over time.

Alternatively, the memory may store a range of potential lipid targets for different patient categories, and the processing unit may select the most appropriate category based on the clinical data of the patient. This allows for a semi-personalized approach in cases where fully personalized data is unavailable or incomplete.

The output of this computation is a set of patient-specific target lipid ranges, which may be updated dynamically as new clinical data becomes available or as guidelines in the memory are updated with new medical findings.

It is noted that in some embodiments, the processing unit is further configured to receive a pharmacogenetic analysis of the patient. The pharmacogenetic analysis refers to the genetic profiling of the patient, which may include, but is not limited to, data on single nucleotide polymorphisms (SNPs), gene expression levels, and other genetic markers that influence the metabolism and efficacy of lipid-lowering medications.

Preferably, the system integrates this pharmacogenetic analysis to adjust lipid-lowering medications according to individual genetic determinants. These genetic determinants may affect how the patient metabolizes certain drugs, such as statins, PCSK9 inhibitors, ezetimibe, or other lipid-lowering agents. By analysing the pharmacogenetic data, the system may optimize drug selection and dosing to enhance therapeutic efficacy, ensuring that the prescribed medications align with the patient's genetic makeup.

More preferably, the system is designed to avoid mild and severe adverse effects by identifying genetic variations that may predispose the patient to adverse drug reactions. For example, certain genetic variants may make the patient more susceptible to statin-induced myopathy, hepatotoxicity, or other side effects. Therefore, in some embodiments, the system may adjust medication choice or dosage to mitigate these risks, improving patient safety and reducing the likelihood of discontinuation due to side effects.

In certain preferred embodiments, the pharmacogenetic analysis may be performed by external genetic testing platforms and transmitted to the clinical decision support system, where it is processed and interpreted in the context of the patient's overall clinical profile. The system may also store predefined thresholds for drug safety and efficacy based on genetic data, enabling real-time comparison and decision-making.

Alternatively, the system may utilize machine learning models trained on large pharmacogenetic datasets to predict the likelihood of adverse effects for specific medications and suggest alternative treatments if a higher risk is identified. The adjustments to lipid-lowering medications based on pharmacogenetic data may also account for potential drug-drug interactions and patient-specific metabolic pathways.

In addition, the pharmacogenetic data may be updated over time, allowing the system to refine its medication adjustments as more genetic information becomes available or as new therapies are developed.

In some embodiments, the pharmacogenetic analysis comprises creating a pharmacogenetic profile based on the 5SPM (5 Step Precision Medicine) model, which allows to identify pharmacogenetic and pharmacologic interactions to direct the treatment. This model has proven a reduction in adverse effects, morbimortality and is more cost effective when applied to patients under multiple medications.

It is noted that the processing unit is further configured to receive measured lipid profile values from blood analytics of the patient. The measured lipid profile values may include, but are not limited to, levels of LDL-C, HDL-C, the ratio LDL-C/HDL-C, the total cholesterol, triglycerides, and other biomarkers relevant to cardiovascular risk assessment.

The system may receive this data from various sources, such as laboratory reports, point-of-care testing devices, or remote monitoring systems that transmit the blood analytics to the clinical decision support system. These lipid measurements may be updated periodically, depending on the clinical protocol or the patient's treatment schedule. The data may be integrated into the system either through manual entry by healthcare providers or through automated data exchange with healthcare information systems, ensuring real-time updates of the patient's lipid profile.

It is further noted that the processing unit is further configured to compare the measured lipid profile values with the personalized target lipid profile values. This comparison is intended to assess whether the patient's current lipid levels fall within the personalized target ranges or if there is any deviation.

The comparison may involve checking each lipid parameter individually (e.g., LDL-C, HDL-C), an average of any combination of measured lipid profile values, a parameter obtained as a function of the computed and measured lipid profile values, and/or comparing a representative parameter or lipid profile value, among other options, against its respective target range, preferably taking into account the personalized adjustments based on the patient's clinical data and more preferably taking into consideration as well the pharmacogenetic profile. The system may calculate the degree of deviation for each lipid parameter or profile value or any combination thereof, which could be expressed as a percentage or an absolute difference between the measured values and the target values.

According to one or more preferred embodiments of the invention, the comparison may trigger a real-time alert or notification if significant deviations from the target lipid profile values are detected. These alerts may prompt healthcare providers to take action, such as adjusting the patient's medication or recommending lifestyle modifications.

Alternatively, the system may be configured to monitor trends over time, comparing consecutive lipid profile measurements to detect gradual changes in lipid levels, which could indicate either improvement or worsening of the patient's lipid management. This longitudinal tracking provides a more dynamic and responsive approach to adjusting the patient's treatment plan based on the evolving clinical picture.

In addition, the system may store historical lipid profile comparisons in the memory, allowing healthcare providers to review past data and make informed decisions about long-term treatment strategies.

It is noted that the processing unit, if one or more measured lipid profile values of the patient lies outside the corresponding personalized ranges of target lipid profile values, is further configured to compute a list of potential medication treatments, aimed at correcting the deviation in lipid levels. The list of medication treatments is sorted based on one or more, two or more, three or more, or all of the following factors: the estimated effect of each medication treatment on the lipid profile values, the difference between the measured lipid profile values and the personalized ranges of target lipid profiles values, at least part of the clinical data of step, and the pharmacogenetic analysis.

The estimated effect of each medication treatment may take into account clinical data from prior studies, patient-specific clinical data from step (a), and the pharmacogenetic analysis from step (c). The estimated effect of each medication is calculated by considering how effectively it can reduce or adjust the specific lipid parameters that are outside the personalized target ranges, such as LDL-C, HDL-C, total cholesterol, or triglyceride.

Additionally, the system may consider the degree of deviation between the measured lipid profile values and the personalized target ranges when generating and sorting the list of medication treatments. For instance, medications that have a greater potential to address larger deviations may be ranked higher on the list or contribute more to the final sorting. For instance, if the patient's LDL-C level is significantly above the personalized target range, lipid-lowering agents with stronger LDL-C reduction properties may be prioritized in the ranked list.

The system may also incorporate at least part of the clinical data received into the computation. This may comprise, but is not limited to, factors such as the patient's medical history, existing comorbidities, and any other relevant physiological or biometric data that may influence the choice of medication. For example, certain medications may be contraindicated for patients with particular health conditions, and this information is factored into the ranking process to avoid recommending treatments that may pose additional risks.

Preferably, the pharmacogenetic analysis is also employed in the computation of the list and in the sorting of the medication treatments thereof. The system may prioritize medications that are more likely to be effective based on the patient's genetic profile and metabolism. Additionally, the system preferably aims to avoid medications that could trigger mild or severe adverse effects, as identified through the pharmacogenetic data. This personalized approach ensures that the treatment options are not only effective but also safer for the individual patient.

In some embodiments, the list of medication treatments may also account for potential drug-drug interactions if the patient is already prescribed other medications. The system may cross-reference the clinical data received with the pharmacogenetic analysis and any existing drug therapies to ensure that the recommended treatments do not negatively interact with the patient's current regimen.

Alternatively, the system may allow healthcare providers to manually adjust the weighting of certain factors, such as the importance of minimizing adverse effects or achieving rapid lipid level correction, thereby providing a customizable treatment ranking.

It is noted that the processing unit is further configured to output data related to various aspects of the patient's lipid management. This output may comprise, but is not limited to, data concerning the personalized target lipid profile values computed, the comparison or the difference between the measured lipid profile values and the personalized target, and the sorted list of medication treatments.

Therefore, in some embodiments the system is configured to output data related to the personalized target lipid profile values, the comparison or the difference of the measured lipid profile values of the patient with the personalized ranges of target lipid profile values, and/or the sorted list of medication treatments, preferably outputs at least data related to the sorted list of medication treatments, more preferably outputs data related to the personalized target lipid profile values, the comparison or the difference of the measured lipid profile values of the patient with the personalized ranges of target lipid profile values, and the sorted list of medication treatments.

This information may be presented in a format that is easily interpretable by clinicians, such as numeric ranges or graphical representations, showing the target lipid levels for key parameters like LDL-C, HDL-C, and triglycerides.

The output of the comparison or difference between the patient's measured lipid profile values and the personalized target ranges may be displayed as an absolute value or percentage deviation for each lipid parameter, making it easier for clinicians to assess the severity of the deviation. In some embodiments, this output could be visualized in a report format, showing historical trends over time, helping to identify whether the patient's lipid profile is improving, worsening, or remaining stable.

Additionally, the output preferably comprises the sorted list of medication treatments ranked based on the estimated effect on the lipid profile values, the clinical data, and/or pharmacogenetic analysis, among other options. The list may be accompanied by detailed information on each medication, such as its predicted impact on specific lipid parameters, potential side effects, and contraindications based on the patient's genetic data or current medication regimen.

In some embodiments, the system may output this data in a customizable format, allowing healthcare providers to select which aspects of the patient's lipid management they wish to view or prioritize. For example, the output could emphasize lipid deviations that require urgent attention or rank medications by different criteria, such as efficacy or safety.

The output may be presented through various interfaces, such as printed reports, digital dashboards, laptops, tablets, TVs, computers, or integrated directly into electronic health record (EHR) systems. Alternatively, the system may support export functionality, enabling the data to be shared with other healthcare professionals or systems, facilitating collaborative decision-making and comprehensive patient care.

According to one or more embodiments, the system may also be configured to output data directly to the patient through a patient-facing application or portal, providing transparency into their lipid profile and treatment plan. This could enhance patient engagement and adherence by keeping them informed of their health status and treatment progress.

Advantageously, the clinical decision support system described herein provides a comprehensive and personalized approach to managing lipid disorders, overcoming the limitations of current standardized treatment protocols. By receiving and integrating detailed patient-specific clinical data, comprising medical records, physiological data, and/or prescribed medication data, the system allows for highly individualized lipid-lowering strategies. Additionally, in some embodiments, pharmacogenetic analysis are also taken into consideration to generate a personalized treatment approach that ensures that lipid profile targets are tailored to each patient's unique clinical characteristics, improving the likelihood of achieving optimal lipid control and reducing the risk of adverse cardiovascular events.

Furthermore, the system may dynamically adjust the treatment recommendations by comparing the patient's measured lipid levels with personalized target values based on established medical guidelines. This monitoring enables prompt identification of deviations from target ranges, providing healthcare providers with timely and actionable insights. The system's ability to compute and rank medication options based on the estimated therapeutic effect, clinical data, deviation from target values, and/or the pharmacogenetic profile further ensures that the selected treatment is not only effective in correcting lipid imbalances but also minimizes the risk of drug-related side effects. This integration of genetic information into the treatment decision process represents a significant improvement over current alternatives, which often overlook individual genetic determinants.

Additionally, the system's capability to output data related to the patient's personalized lipid targets, lipid deviations, and ranked treatment options enhances both clinical decision-making and patient engagement. Healthcare providers can make informed, data-driven decisions faster, improving patient outcomes. The system's adaptability, including its potential integration into electronic health records and patient-facing platforms, allows for an integral healthcare management, improving adherence to treatment regimens and further mitigating the risk of cardiovascular events.

In some preferred embodiments of the invention, the lipid profile of the CDS system of the invention comprises LDL cholesterol, total cholesterol (TC), LDL cholesterol, triglycerides, HDL cholesterol, VLDL cholesterol, remnant cholesterol particles, the ratio of TC/HDL cholesterol, the ratio LDL/HDL cholesterol, lipoprotein (a), apolipoprotein A1, apolipoprotein B, and/or non-HDL cholesterol levels, preferably comprises LDL cholesterol, more preferably comprises LDL cholesterol as the main input tool or main parameter.

It is noted that the system may comprise a comprehensive set of lipid profile markers beyond just LDL cholesterol or the above mentioned parameters, and may incorporate additional key biomarkers that offer a more complete assessment of cardiovascular risk. LDL cholesterol is preferably the primary input, given its critical role in atherogenesis and its established importance in lipid management strategies. Total cholesterol (TC) and HDL cholesterol provide further context, for example by evaluating the balance between "bad" and "good" cholesterol, while the TC/HDL ratio offers a more holistic risk indicator.

Triglycerides and VLDL cholesterol may contribute valuable information on the lipid content carried by other lipoproteins, particularly in relation to metabolic syndrome or diabetes, conditions frequently associated with elevated cardiovascular risk. The inclusion of remnant cholesterol particles, which are remnants of VLDL and intermediate-density lipoproteins, provides additional granularity, as these particles have been increasingly recognized for their role in atherosclerosis.

Lipoprotein (a) and apolipoproteins A1 and B provide critical genetic and structural insights. Lipoprotein (a) is a genetically determined risk factor for atherosclerosis, and its measurement offers an important tool for identifying patients at elevated cardiovascular risk independent of LDL-C levels. Apolipoprotein B, as the primary protein in atherogenic lipoproteins, may serve as a reliable indicator of the total number of potentially harmful lipoproteins, whereas apolipoprotein A1 is the main protein of HDL particles and is used to assess the efficiency of reverse cholesterol transport.

Non-HDL cholesterol levels may be comprised to represent all atherogenic lipoproteins, providing a more comprehensive measure of cardiovascular risk beyond just LDL-C. This is particularly useful in cases where triglyceride levels are elevated, as non-HDL cholesterol captures the full range of cholesterol-carrying particles contributing to atherosclerosis.

Advantageously, the inclusion of tone or more of these lipid profile markers allows the CDS system of the invention to offer a more detailed and accurate cardiovascular risk assessment than, for instance, systems focused solely on LDL cholesterol. By analysing a broader set of biomarkers, the system can provide a more nuanced understanding of the patient's lipid metabolism, identify additional risk factors, and tailor treatment recommendations more precisely. This comprehensive approach enhances the system's ability to predict cardiovascular events and guide treatment adjustments, improving overall patient outcomes and addressing individual patient variability more effectively than current systems based on limited lipid measurements.

In some preferred embodiments of the invention, the pharmacogenetic analysis of the CDS system of the invention comprises data on the tolerance of the patient towards dyslipidaemia medication and/or data related to the genetic predisposition and individualized response of the patient towards a particular lipid profile.

It is noted that the system may comprise pharmacogenetic data that specifically addresses the patient's tolerance to lipid-lowering medications. This tolerance data may include genetic markers that predict the likelihood of adverse reactions, such as statin-induced myopathy, liver toxicity, or other medication-related side effects. By analysing the patient's genetic predisposition to tolerate certain medications, the system can recommend treatments that are less likely to cause side effects, thereby improving adherence and reducing treatment discontinuation.

Genetic variations may influence not only the metabolism of lipid-lowering medications but also the patient's natural lipid levels and the efficacy of certain treatments in achieving target lipid ranges. For example, certain patients may have genetic polymorphisms affecting LDL receptor function or hepatic lipase activity, leading to higher baseline LDL-C or triglyceride levels and requiring tailored treatment approaches. In some embodiments the system adjusts its recommendations based on these genetic determinants to optimize therapeutic outcomes for each individual patient.

According to one or more preferred embodiments of the invention, the pharmacogenetic analysis may also include predictive data on how the patient's lipid profile is likely to evolve over time based on genetic predispositions, allowing for more proactive management of dyslipidaemia. The system may integrate this genetic information with other clinical data to provide a comprehensive, personalized treatment plan.

Advantageously, by incorporating data on both the patient's tolerance to dyslipidaemia medications and/or their genetic predisposition towards lipid profiles, the system ensures a highly personalized approach to lipid management. This enhances the safety and efficacy of treatment by selecting medications that not only target lipid abnormalities effectively but are also well-tolerated by the patient. As a result, the system minimizes the risk of adverse drug reactions, improves long-term adherence to therapy, and increases the likelihood of achieving optimal lipid control. This level of personalization is a significant improvement over current systems that do not fully account for the genetic variability in medication response and tolerance.

In some preferred embodiments of the invention, the clinical data of the CDS system of the invention comprises data related to the adherence of the patient to the prescribed medication, preferably wherein the processing unit of the system is further configured to output data related to the adherence of the patient to the prescribed medication, more preferably is further configured to output data related to the adherence of the patient to the prescribed medication if the measured lipid profile of the patient diverges from the personalized target lipid profile.

It is noted that the system may receive and process data concerning the patient's adherence to prescribed lipid-lowering medications. This data may comprise information on medication refill patterns, reported usage, and/or data from smart pill dispensers or wearable devices that track medication intake, among other options. By integrating adherence data into the clinical decision support system, healthcare providers can more accurately determine whether deviations in the patient's lipid profile are due to poor adherence or other factors such as treatment inefficacy or genetic variability.

When the measured lipid profile values deviate from the personalized target lipid ranges, the system is, in some embodiments, configured to output specific data related to the patient's adherence. This output may highlight whether non-adherence is a likely cause of the lipid level deviations, enabling healthcare providers to address the issue directly. For example, the system may indicate patterns of missed doses or irregular medication usage that correlate with suboptimal lipid control, helping to differentiate between adherence-related and treatment-related problems.

According to one or more preferred embodiments of the invention, the system may provide adherence alerts or notifications to both healthcare providers and patients, encouraging timely interventions. This feature allows for a more precise and responsive approach to managing dyslipidaemia, ensuring that treatment plans are adjusted based on the actual adherence behaviour of the patient.

Advantageously, the inclusion of adherence data enhances the system's ability to provide accurate and personalized treatment recommendations. By distinguishing between deviations in lipid levels caused by poor adherence versus those caused by treatment failure or genetic factors, the system enables healthcare providers to make more informed decisions about modifying or intensifying therapy. This improves patient outcomes by addressing the root causes of suboptimal lipid control, reducing unnecessary medication changes, and promoting better long-term adherence to prescribed treatments.

In some preferred embodiments of the invention, the clinical data of the CDS system of the invention comprises risk factors, preferably wherein the computation of the personalized target lipid profile further takes said risk factors as input.

These risk factors may comprise, but are not limited to, age, gender, smoking status, presence of comorbidities such as diabetes or hypertension, family history of cardiovascular disease, obesity, and lifestyle factors such as physical activity levels or diet. By incorporating these risk factors, the system ensures that the personalized lipid profile target is not solely based on static guidelines but is adapted to the patient's broader cardiovascular risk profile.

For example, patients with higher cardiovascular risk, such as those with a history of heart disease or multiple comorbidities, may have more aggressive target lipid levels (e.g., lower LDL-C targets) to reduce their risk of adverse events. Conversely, patients with fewer risk factors may be given more moderate lipid targets. According to certain embodiments, the system calculates these personalized ranges by considering how each risk factor impacts the overall cardiovascular risk, ensuring that treatment intensity is aligned with the patient's individual risk profile.

In some other preferred embodiments of the invention, the system may also dynamically adjust the target lipid profile ranges as risk factors change over time. For instance, if a patient quits smoking or achieves significant weight loss, the system may adjust the lipid targets to reflect the reduced cardiovascular risk. This adaptability ensures that the treatment goals remain relevant and personalized throughout the patient's management plan.

Advantageously, by incorporating risk factors into the computation of the personalized target lipid profile, the system provides a more accurate and individualized approach to lipid management. This improves the system's ability to tailor treatment strategies to the patient's overall cardiovascular risk, ensuring that high-risk patients receive appropriately aggressive treatment while avoiding overtreatment in lower-risk individuals. This targeted approach helps to optimize treatment efficacy, reduce unnecessary medication burden, and ultimately lowers the risk of cardiovascular events.

In some preferred embodiments of the invention, a risk factor of the CDS system of the invention is the prevalence or not of diabetes in the patient and the type of diabetes.

The system may distinguish between different types of diabetes, including type 1 and type 2 diabetes, as well as other diabetes-related conditions such as gestational diabetes or prediabetes. Each of these conditions carries distinct implications for cardiovascular risk, and the system adjusts the target lipid levels accordingly.

Patients with diabetes, particularly those with type 2 diabetes, are at a heightened risk for cardiovascular events due to the combined impact of hyperglycaemia, insulin resistance, and often accompanying dyslipidaemia. In these patients, more stringent lipid targets-such as lower LDL-C levels-may be required to mitigate their elevated cardiovascular risk. For instance, patients with type 2 diabetes are commonly recommended to achieve an LDL-C level lower than the general population to reduce the likelihood of atherosclerotic events.

Advantageously, by integrating diabetes status and its specific type as a risk factor, the system enhances the precision of the personalized lipid management plan. This ensures that patients with diabetes, who face unique cardiovascular risks, receive treatment strategies that are tailored to their metabolic profile and overall cardiovascular condition. As a result, the system improves the effectiveness of lipid-lowering interventions in diabetic patients, reducing the incidence of cardiovascular complications while ensuring individualized care.

According to one or more preferred embodiments of the invention, a risk factor of the CDS system of the invention is the existence of a medical record related to one or more previous atherothrombotic events, coronary heart diseases, peripheral arterial diseases, and/or cerebral ischemia, preferably wherein said medical record is a system input point.

It is noted that in some embodiments the system integrates a history of atherothrombotic events or related cardiovascular conditions as a significant risk factor when computing the personalized target lipid profile. These conditions may comprise, but are not limited to, prior myocardial infarction, stroke, peripheral arterial disease, and other manifestations of atherosclerosis. A medical history of such events dramatically increases the patient's risk for future cardiovascular incidents, necessitating more aggressive lipid-lowering strategies.

In some preferred embodiments of the invention, a patient's medical record with information regarding these previous cardiovascular conditions is used as a system input point. This may comprise automated data retrieval from electronic health records (EHRs) or manual input by healthcare providers. By including these relevant medical data points, the system can better assess the patient's overall cardiovascular risk and adjust the lipid profile targets accordingly. For instance, patients with a history of coronary artery disease may be assigned lower LDL-C targets to reduce the probability of future atherothrombotic events.

It is further noted that the system may also adjust treatment recommendations dynamically based on the recurrence or progression of these conditions. If a patient has had multiple atherothrombotic events, the system may recommend an even more aggressive lipid-lowering treatment plan, taking into account the increased risk posed by cumulative cardiovascular damage.

Advantageously, by including previous atherothrombotic events and related cardiovascular conditions as risk factors, the system provides a highly tailored and risk-sensitive approach to lipid management. Patients with a history of such conditions require more intensive monitoring and treatment, and the system ensures that the target lipid profiles and recommended treatments reflect this heightened risk. This leads to more precise interventions aimed at preventing recurrent events, improving long-term cardiovascular outcomes for high-risk patients.

In some preferred embodiments, more than one measured lipid profile from blood analytics of the patient is received together with a time reference or the date of said blood analytics, wherein the processing unit of the system is configured to perform the following step: compare the last measured lipid profile values of the patient with the personalized ranges of target lipid profile values, wherein if said last measured lipid profile values of the patient lies outside the personalized ranges of target lipid profile values, then provide a list of medication treatments wherein said list is sorted based on one or more, two or more, three or more, four or more, or all of the following aspects: the evolution of the measured lipid profiles values with time, the effect of each medication treatment on the lipid profile of the patient, the difference between the measured lipid profile values and the personalized ranges of target lipid profile values, at least part of the clinical data, and/or the pharmacogenetic analysis. Preferably the list is sorted at least based on the evolution of the measured lipid profiles values with time.

It is noted that in some embodiments the system may be capable of receiving and analysing multiple lipid profile measurements over time, with each measurement being associated with a specific time reference or date. This allows the system to track the evolution of the patient's lipid levels and observe trends that could inform treatment decisions. By comparing the last measured lipid profile with the personalized target ranges, the system evaluates the most recent status of the patient's lipid levels, focusing on whether they lie outside the target ranges.

If deviations from the target ranges are identified, according to some embodiments the system generates a list of medication treatments, wherein the list may be prioritized based on the historical evolution of the patient's lipid levels. This allows the system to consider whether the patient's lipid levels have been consistently worsening, improving, or fluctuating, and to recommend treatments that are most likely to reverse negative trends or maintain positive progress.

In some preferred embodiments, the system may factor in the rate of lipid level changes when sorting the medication list, giving priority to treatments that can either rapidly address significant deviations or stabilize the patient's lipid profile if there are gradual fluctuations. Additionally, the system may continue to incorporate the effect of each medication treatment, clinical data such as comorbidities and existing medications, as well as pharmacogenetic analysis, ensuring that the treatment options are personalized and optimized for the patient's unique profile.

Advantageously, the ability to analyse multiple lipid measurements over time and base treatment decisions on the patient's lipid profile evolution provides a dynamic and responsive approach to managing dyslipidaemia. This temporal dimension allows healthcare providers to make more informed decisions by considering not only the current lipid levels but also the trajectory of the patient's lipid control. As a result, the system improves the precision and timeliness of treatment adjustments, helping to stabilize lipid levels more effectively and reduce the risk of adverse cardiovascular events.

In some preferred embodiments of the clinical decision support system of the invention, the system comprises a display configured to display the output data.

It is noted that said display may be computer screen, a tablet, a phone, a laptop, extended reality headset, and/or any other suitable electronic medium comprising a screen. Alternatively, the output data may be displayed by a projector.

In some preferred embodiments, the clinical decision support system of the invention comprises the memory above referenced. Alternatively, the CDS system may be in communication with an external cloud server comprising said memory.

According to certain preferred embodiments, the data related to medication currently prescribed to the patient, and/or the list of medication treatments provided by the CDS system of the invention, may comprise dyslipidaemia medication, hypolipidemic medication and/or anti-hypolipidemic medication, such as, but not limited to, statins, such as Atorvastatin, Simvastatin, Rosuvastatin, Pravastatin and/or Lovastatin, fibrates, such as Bezafibrate, Gemfibrozil and/or Fenofibrate, bile acid sequestrants, such as Cholestyramine, Colestipol and/or Colesevelam, cholesterol absorption inhibitors, such as Ezetimibe and/or Ezetimibe/simvastatin, niacin derivatives, omega-3 fatty acid supplements, PCSK9 targeted therapy, such as Alirocumab, Evolocumab or Inclisiran, and/or Bempedoic acid.

In one or more preferred embodiments of the invention, it is received in the CDS system of the invention at least medical records and medication currently prescribed to the patient as clinical data, and wherein if the medical records comprises certain current medical conditions, and the medication currently prescribed to the patient comprises lipid-lowering medication, then the processing unit of the system is further configured to output a safety warning indicating possible secondary effects of the medication. It is noted that preferably said current medical conditions are at least one of myalgia, rhabdomyolysis and/or high transaminases. Additionally, in some embodiments said current medical conditions may comprise at least one of Polymitositis, Dermatomytositis, liver diseases, hypothyroidism, chronic kidney disease and mitochondrial myopathies.

Advantageously, this allows to prevent create or worsen secondary effects that may be associated with the lipid-lowering medication currently prescribed, therefore providing invaluable information to avoid worsening or creating a medical condition and approach the lipid-lowering goals from a different angle, such as by providing different medication that do not have those possible secondary effects.

In one or more preferred embodiments of the CDS system of the first aspect of the invention, the personalized ranges of target lipid profile comprises at least one first threshold subrange, or an additional range, wherein if at least one value of the measured lipid profile values of the patient is inside said at least one threshold subrange, and the patient does not have currently any dyslipidaemia medication prescribed, then do not provide a list of medication treatments and instead output a recommendation of healthy lifestyle.

It is noted that said ranges and subranges may be defined such that they do or do not overlap, wherein, if overlapping, it may be given priority to one or other range or subrange. Preferably, giving priority to the subrange or subranges.

Therefore, if the measured lipid profile of the patient has values inside these subranges, that would be indicative of a deviation from calculated or recommended personalized ranges of target lipid profile values small enough to be corrected with a healthier lifestyle, instead of passing directly to medication. Therefore, preferably said at least one threshold subrange is close to the computed personalized ranges of target lipit profile values or recommended values according to one or more medical guidelines, or is overlapping and/or or adjacent, whereas the range that triggers a medication recommendation extends beyond said subrange into measured lipid profile values which are further from the calculated target or recommended ones.

Advantageously, this way unnecessary medication prescription is avoiding, preventing thus the possibility of experiencing secondary effects, and reducing the cost. Not only that, this approach has a synergic positive effect with other potential medical conditions, as this may push the patient to adopt a healthier lifestyle with long-lasting impact in every aspect of their lives.

In one or more preferred embodiments of the CDS system of the first aspect of the invention, the personalized ranges of target lipid profile comprises at least one second threshold subrange, wherein if at least one value of the measured lipid profile values of the patient is inside said at least one threshold subrange, and the patient currently has one or more dyslipidaemia medication prescribed, then output a recommendation of healthy lifestyle and provide a list of medication treatments, preferably sorted based on one or more, two or more, three or more or all of the following factors: the effect of each medication treatment on the lipid profile values of the patient, the threshold subrange in which the measured lipid profile lies, at least on part of the clinical data, and/or the pharmacogenetic analysis.

For example, said second subthreshold may comprise values that diverge more from the recommended computed personalized values than the first subthreshold, therefore, additionally to a healthier lifestyle, it would be recommended some medication as well.

Advantageously, this way the CDS system of the invention is adapted to provide intermediate solutions that combine a healthier lifestyle and medication, which can present a positive effect on a particular demographic of the population with, for example, moderate dyslipidaemia.

According to a second aspect of the clinical decision system of the invention (CDS), a computer program is disclosed, wherein said computer program comprises instructions which, when executed by a processor, causes the processor to carry out the steps comprised in the configuration of the processing unit of the CDS system according to any embodiment of the first aspect of the invention, further comprising any possible alternative or feature herein described.

According to a third aspect of the clinical decision system of the invention (CDS), a computer readable storage medium is disclosed, wherein said computer readable storage medium comprises instructions which, when executed by a processor, causes the processor to carry out the steps comprised in the configuration of the processing unit of the CDS system according to any embodiment of the first aspect of the invention, further comprising any possible alternative or feature herein described.

To enable a better understanding of the present disclosure, reference will now be made, by way of example only, to the accompanying schematic drawings. It is important to note that the following schematic drawings are provided solely as exemplary, non-limiting potential embodiments of the invention. Accordingly, the drawings are to be regarded as illustrative instead of restrictive, serving merely to aid in the explanation of the invention's principles and potential applications.

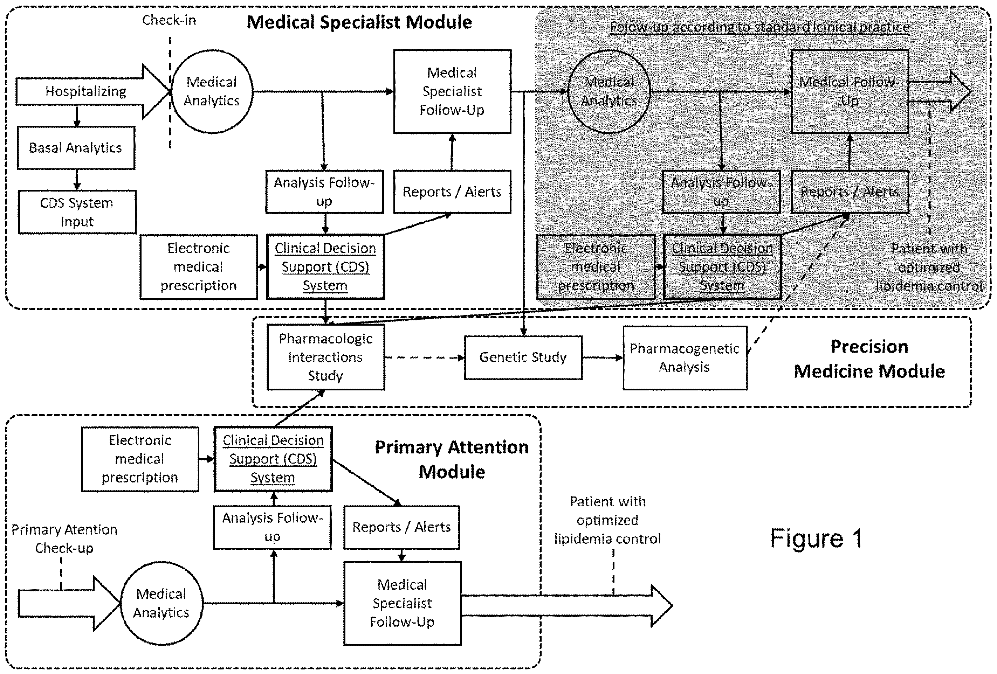

Figure 1 shows a diagram of an example of a lipid management medical process comprising the CDS system according to one or more embodiments of the invention, from the entrance into the medical system point of view (Check-in). In particular, it shows 3 interconnected modules; the medical specialist module, the precision medicine module, and the primary attention module.

Referring to figure 1, the input to the system can be produced by a clinical event in a given patient during hospitalization, in the case of the Medical specialist Module, in which a data entry in the form of a baseline analysis has been performed and which constitutes the input to the decision support system, or it can be produced from a primary attention or rutinary check-up, in the case of the Primary Attention Module, wherein both may be connected to the Precision Medicine Module. The input of data from the prescribed medication after the baseline and follow-up analysis allows, firstly, the issuing of a follow-up alert to the specialist physician and, secondly, the pharmacogenetic study of interactions, which is part of the precision medicine module. Once both steps have been completed, a personalized report of recommendations will be issued, adjusted to age, comorbidities, previous medication and possible interactions based on the pharmacogenetic profile. The performance of successive analyses allows for system feedback and adjustment of the treatment recommendation given.

In the case in which the CDS system is integrated in the medical specialist practice, such as in the case of the Medical Specialist Module, upon Check-in, a patient is admitted to the system, which can occur during hospitalization or another medical event,and the data accompanying this check-in may be comprised in the Basal Analytics, which can be communicated to the CDS System. The first step after check-in is referred to in figure 1 as Medical Analytics, which comprises analysis and obtaining the results, such as for blood analysis, among other options. This analysis, the corresponding follow-up, the Basal Analytics and information regarding electronic medical prescriptions of the patient constitutes the input into the Clinical Decision Support (CDS) System according to one or more embodiments of the invention, which can therefore use this data to issue medication recommendations or trigger reports/alerts, which eventually are communicated to the Medical specialist, who presumably also gets communication of the original medical analysis.

As it can be seen in the shaded region, this process may be repeated, wherein the original check-in is substituted by a follow-up medical analytics, which can again be introduced in the CDS system of the invention, together with information of electronic medical prescriptions, to recommend possible medications, elaborate reports and/or create alerts that will show up to the clinician in-situ or upon subsequent medical follow-ups.

Additionally, the CDS system if the invention preferably is in communication with the precision medicine module, which leverages information such as the Pharmacologic Interactions Study, and genetic studies, to elaborate a Pharmacogenetic analysis which can be used by the CDS system of the invention to elaborate or modify the medication recommendations, the reports and/or the alerts based on said pharmacogenetic study.

Parallel to the Medical Specialist Module, the Primary Attention Module serves as an entry point for patients who are not hospitalized but require primary care. Upon Check-up, similar to the specialist module, patient data is collected via Medical Analytics. This data can be fed into the CDS System, which also receives information from Electronic Medical Prescriptions apart from information from the medical analytics, and, preferably, also from the pharmacogenetic analysis from the Precision Medicine Module, to elaborate a medication recommendation based on said information, and generate reports and/or alerts for the Medical Specialist follow-up.

The system allows for continuous feedback from medical information of the patient, prescribed medicine and related treatment adherence information, analytics (such as blood and/or urine analytics) and pharmacogenetic analysis, and allows to adjust treatment over time, considering age, comorbidities, previous treatments, and genetic profiles.

In some embodiments the system can also be adapted to process information from other conditions where pharmacogenetic interactions are critical, including conditions such as hypertension or diabetes, wherein personalized medicine and genetic profiling play an important role.

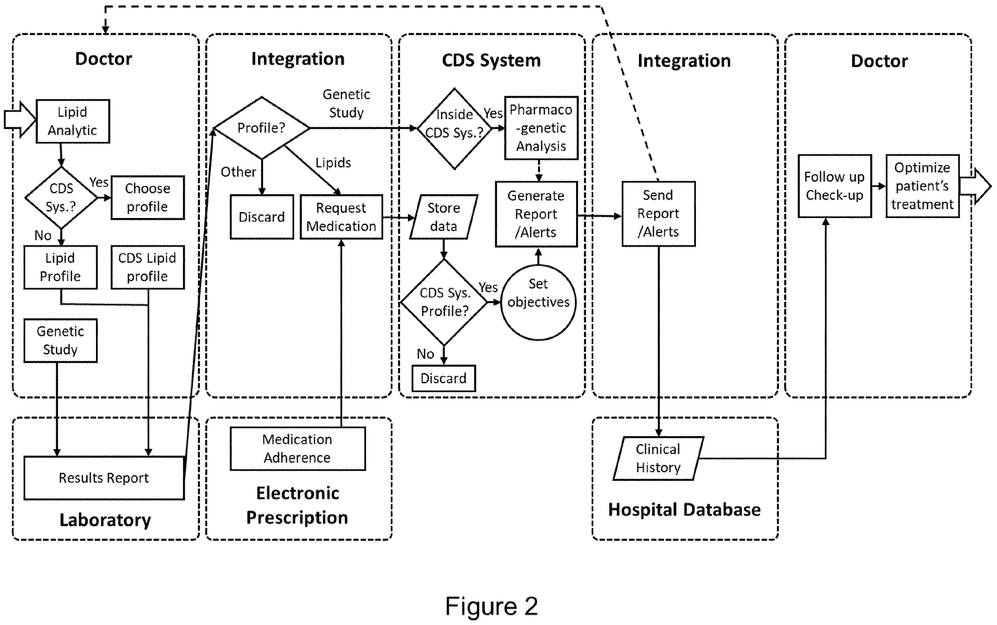

Figure 2 shows a flow chart of an example of the integration of the CDS system, according to one or more embodiments of the invention, in a lipid management medical process. The different modules displayed represent the doctor, the laboratory, electronic prescriptions and medical history databases, and their integration with the CDS system of the invention.

The integration starts from the usual clinical practice by means of the initial analytical request. As can be seen, the integration is carried out at the level of electronic prescription, laboratory data obtained from the corresponding repositories and the patient's digitalized medical history. The lipid profile that triggers the recommendation system is specific, avoiding the emission of recommendations in other cases. If the profile is already in the system and significant adverse clinical or analytical events are found, the pharmacogenetic study can be started automatically. All this leads to an early and personalized optimization of lipid-lowering medication.

Regarding the Clinical Decision Support system from Figure 2, the CDS system of the invention preferably interfaces with various entities, which can be referred to as modules in figure 2, and comprise the Doctor, the Laboratory, Electronic Prescriptions, and a Hospital Database, which integrate or interface with the CDS System itself. The flowchart from figure 2 outlines the process by which patient data is analysed and treated within the framework of both routine clinical practice and depending on the information provided or the use or not of the CDS System. The flow culminates in the optimization of the patient's treatment. The following is a detailed description of each element and its integration into the overall process: the Doctor Module comprises the Lipid Analytics, wherein the process begins with the doctor performing an initial lipid analysis. The doctor, or the CDS system automatically, evaluates whether there a lipid profile needs to be generated in CDS system, or if it already exists (and can be chosen) or can be manually created. Additionally, if necessary, the doctor may initiate a Genetic Study, which offers insights into how the patient's genetic makeup may affect their response to lipid management treatment. This data from the genetic profile and the CDS Lipid Profile is transferred to the laboratory for processing. The Laboratory receives the data inputs (such as the lipid profile and optionally the genetic study results) and provides that information through the Results Report to the CDS System via the Integration Module to serve as information to elaborate a profile. If a lipids profile is elaborated, the CDS system can provide medication recommendations and storing said data, wherein information regarding the adherence to medication is used to provide such medication recommendations which can be requested by the doctor. If the Genetic study is going to be used with the patient information such as the lipids profile, the CDS system checks if said information has been provided to the CDS system and elaborates a pharmacogenetic analysis, which will be provided to the CDS system to take said pharmacogenetic analysis in consideration when elaborating the medication recommendations, reports and/or alerts. Therefore, the lipids profile generated by the CDS system allows to set objectives, which will be used to elaborate medication recommendations, reports and/or alerts together with the pharmacogenetic analysis, if it exists. In some embodiments said recommendations are provided without needing the pharmacogenetic analysis. Said reports, which may comprise medication recommendations, and/or alerts can be sent to the doctor (Integration module again) and also provided to the clinical history of the patient in the hospital's database. When the patient is doing a follow-up check-up with the Doctor, the doctor, based on said reports and/or alerts can optimize the patient's treatment.

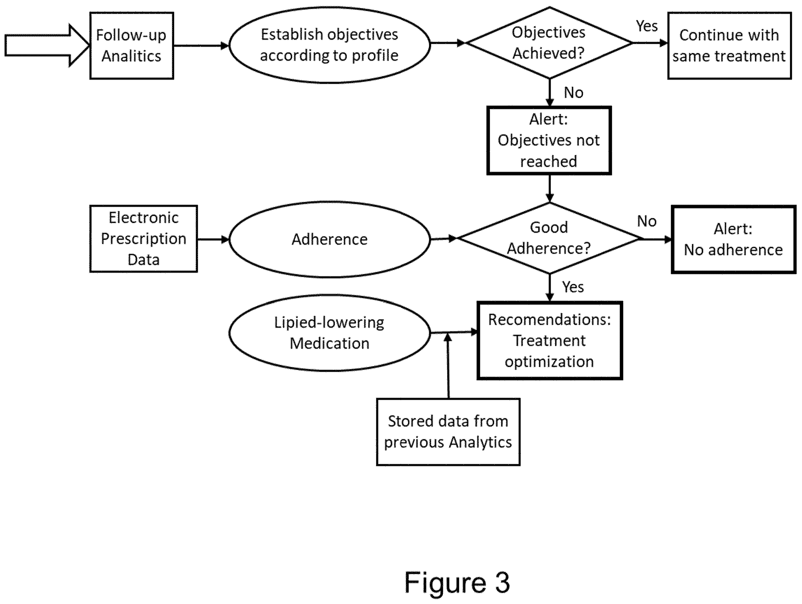

Figure 3 shows a schematic diagram of the logic steps performed by a CDS system according to one or more embodiments of the invention.

According to the exemplary diagram of Figure 3, in some embodiments the follow-up analysis, based on the requested profile, establishes personalised LDL-C targets in accordance with the clinical practice guidelines. If this level is above the threshold, an alert will be issued, which will first check the patient's percentage adherence and issue an adherence recommendation. If this is adequate, it will issue a recommendation for further optimisation based on the baseline analysis stored in the system, the follow-up analysis and the estimated reduction in medication currently being taken by the patient based on the electronic prescription.

The schematic diagram from Figure 3 outlines the logic steps performed by a Clinical Decision Support (CDS) system within a lipid management process, specifically aimed at optimizing treatment based on follow-up analysis and patient adherence. The process is centred on establishing personalized LDL-C objectives, monitoring patient progress, and making treatment adjustments as necessary. The process begins with the system receiving Follow-up Analytics data, which in some embodiments can be obtained from periodic medical check-ups or laboratory results. Based on the patient's Profile, the system Establishes Objectives, typically LDL-C (low-density lipoprotein cholesterol) targets, in accordance with clinical practice guidelines. These targets are preferably personalized for each patient, considering their specific lipid profile, medical history, and/or genetic factors. The system checks whether the Objectives (LDL-C targets) have been achieved: If Yes, the system concludes that the current treatment is effective, and the patient can Continue with the Same Treatment; if No, indicating that the patient's LDL-C levels are still above the desired threshold, an Alert is generated, stating that Objectives Have Not Been Reached. This alert may prompt further investigation into why the treatment is not working as expected. Once the objectives are found to be unmet, the system can check the patient's Adherence to the prescribed medication regimen. Electronic Prescription Data is reviewed to determine if the patient has been taking their lipid-lowering medication as prescribed. If adherence is Good, the system recognizes that the failure to meet objectives is not due to poor compliance, and instead, Recommendations for Treatment Optimization are generated. If adherence is Not Good, an Alert is issued, notifying the healthcare provider of No Adherence, indicating that the patient's lack of adherence to the prescribed regimen could be the reason for unmet objectives. If patient adherence is confirmed, the system moves to Recommendations for Treatment Optimization. Taking into consideration the current Lipid-lowering Medication the patient is taking and Stored Data from Previous Analytics, which in some embodiments may comprise baseline and follow-up analyses, which helps the system estimate whether a more aggressive or alternative treatment strategy is needed to meet LDL-C targets. The system then integrates this information to suggest adjustments in medication type, dosage, or therapeutic strategies to better optimize the patient's treatment plan, according to some embodiments.

The figures are not intended to be exhaustive or to limit the invention to the precise form disclosed. In particular, different steps, processes, modules or submodules displayed in the figures may be comprised or not, in any combination, in different embodiments of the invention. It should be understood that the invention can be practiced with modification and alteration, and that the invention is limited only by the claims and the equivalents thereof.

### Examples

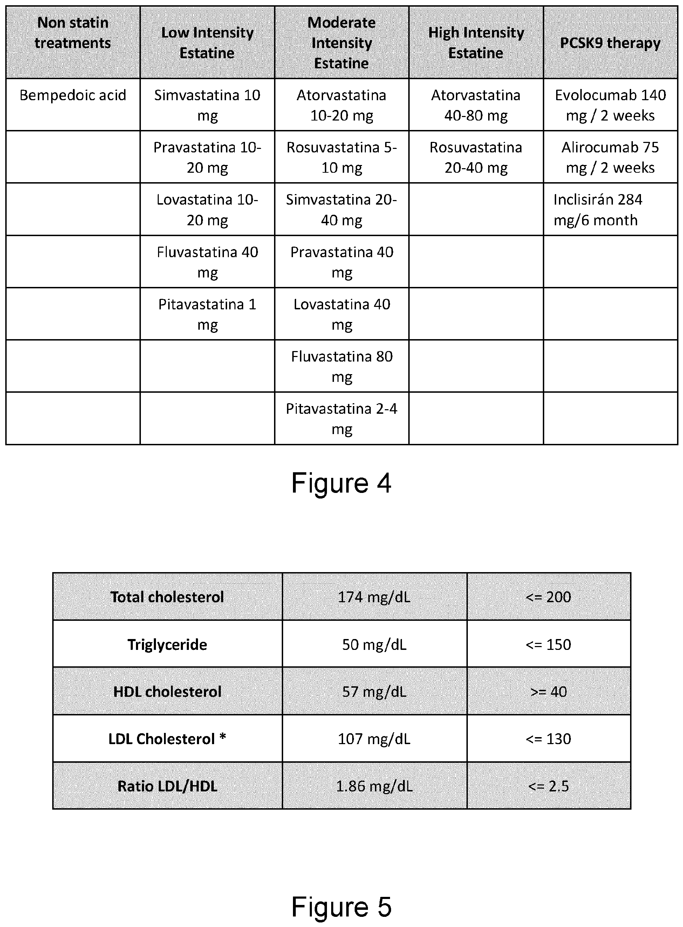

As a way of example, we will describe two situations (Example 1 and Example 2), wherein a system according to one or more embodiments of the invention can be used. In both example we will make use of figures 4 and 5.

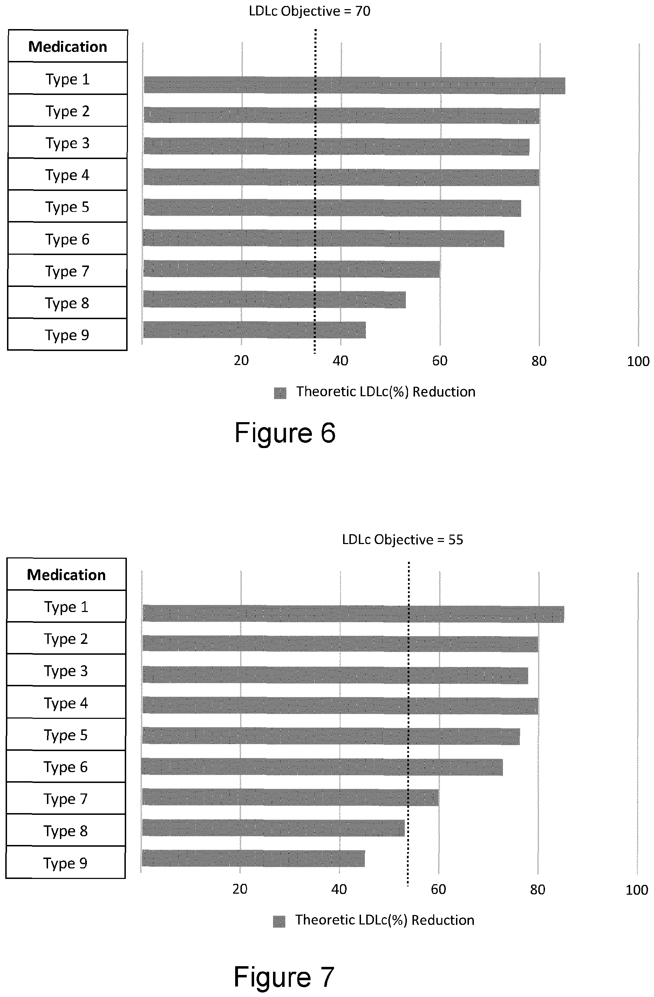

Figure 4 shows a list of medications and corresponding quantities, grouped by intensity or lipid regulation power, together with examples of typically used PCSK9 inhibitors. The medications listed in figures 6 and 7, labelled as Type 1, type 2, etc.. until type 9, refer to different mixes of these medications, wherein the particular composition of each mix is not relevant for the illustrative purposes if these examples. However, each medication type or mix, presents a theroretic LDLc reduction (in percentage) represented as horizontal bars. Figure 5 shows a case example of blood analytics displaying results for cholesterol and triglyceride concentrations in blood, wherein the middle column is the measured value, and the most right column displays basal or standard target ranges, which do not take into consideration a personalized lipid profile (for example, calculated according to one or more embodiments of the invention), and wherein for example "<= 200" means less or equal than 200, or the range [0 - 200].

### Example 1

Figure 6 shows a graph displaying the theoretic LDLc reduction in percentage (as horizontal bats) for each medication type, wherein each medication type is a particular combination of medications from figure 4. The vertical bar denotes the LDLc reduction in percentage required to achieve the objective (in this case, the objective is 70 mg/dL, which for this patient would require around a 35% LDLc reduction) for a specific patient, which in this first example is a patient that comprises particular conditions that diverge from the standard objective values (right-most column of figure 5), and make the personalized LDLc or LDL cholesterol objective value equal or below 70 mg/dL. Said particular conditions may be, for example, being diabetic in high risk of a cardiovascular event, and the target cholesterol range may be derived from a personalized lipid profile according to one or more embodiments of the invention.

This is a practical example of integration where we see a blood test with the basic lipid determinants that become part of the decision support system, establishing a first recommendation of the patient's risk group and LDLc targets for each of the risk levels. Below we can see the different LDLc lowering alternatives that can be ordered from highest to lowest potency and that are aligned with the pharmacotherapeutic strategies of the scientific community.

### Example 2

This case is similar to example 1, with the difference that, referring to figure 7, the LDLc objective now is 55 or lower, which would require an LDLc reduction of around 50-55 percent. This patient presents a stricter or lower LDLc objective range because, for example, it may be a patient that has had any cardiovascular event such as angina, coronary revascularization, peripheric arterial sickness, etc. Therefore, taking in consideration the therapeutic recommendations of the European Cardiology Society of 2019, according to one or more embodiments of the invention, the target lipid profile sets the objective LDLc values at 55 mg/dL or lower.

This is another practical example of integration in a patient with established cardiovascular disease, who requires further LDLc reduction, where we see a blood test with the basic lipid determinants that become part of the decision support system, establishing a first recommendation of the patient's risk level and the LDLc targets determined. Below we can see the different LDLc lowering alternatives that can be ordered from higher to lower potency and that are aligned with the pharmacotherapeutic strategies of the scientific community.

## Claims

1. A clinical decision support system comprising a processing unit in communication with a memory, wherein the processing unit is configured to:
a. receive clinical data associated to a patient, wherein said clinical data comprises medical records, physiological data and/or data related to medication currently prescribed to the patient;
b. compute personalized ranges of target lipid profile values taking as input the clinical data of the patient from step (a) and wherein said personalized target lipid profile values are computed according to medical guidelines values;
c. receive a pharmacogenetic analysis of the patient
d. receive measured lipid profile values from a blood analytics of the patient;
e. compare the measured lipid profile values of the patient from step (d) with the personalized target lipid profile values of step (b);
wherein if one or more measured lipid profile values of the patient lies outside the corresponding personalized ranges of target lipid profile values, then
f. compute a list of medication treatments wherein said list is sorted based on the estimated effect of each medication treatment on the lipid profile values, the difference between the measured lipid profile values and the personalized ranges of target lipid profiles values, at least part of the clinical data of step (a), and the pharmacogenetic analysis of step (c);
wherein the processing unit of the system is further configured to output data related to the personalized target lipid profile values of step (b), the comparison or the difference of the measured lipid profile values of the patient from step (d) with the personalized ranges of target lipid profile values of step (b), and/or the sorted list of medication treatments of step (e).

2. The clinical decision support system according to claim 1, wherein the lipid profile values comprise LDL cholesterol, total cholesterol (TC), Triglycerides, HDL cholesterol, VLDL cholesterol, remanent cholesterol particles, the ratio TC/HDL, the ratio LDL/HDL, lipoprotein (a), apolyprotein A1, apolyprotein B, and/or Non-HDL cholesterol levels, preferably comprises at least LDL cholesterol.

3. The clinical decision support system according to any one of the previous claims, wherein the pharmacogenetic analysis comprises data of the tolerance of the patient towards dyslipidaemia medication and/or data related to the genetic predisposition and individualised response of the patient towards a particular lipid profile.

4. The clinical decision support system according to any one of the previous claims, wherein the clinical data comprises data related to the adherence of the patient to the prescribed medication, and the processing unit of the system is further configured to output data related to the adherence of the patient to the prescribed medication if the measured lipid profile of the patient diverges from the personalized target lipid profile.

5. The clinical decision support system according to any of the previous claims, wherein the clinical data comprises risk factors, and the computation of the personalized target lipid profile further takes said risk factors as input.

6. The clinical decision support system according to claim 5, wherein a risk factor is the prevalence or not of diabetes in the patient and the type of diabetes.

7. The clinical decision support system according to any one of claims 5 to 6, wherein a risk factor is the existence of a medical record related to one or more previous atherothrombotic events, coronary heart diseases, peripheral arterial diseases and/or cerebral ischaemia.

8. The clinical decision support system according to claim 1, wherein more than one measured lipid profiles from blood analytics of the patient are received in step (d), together with a time reference or the date of said blood analytics, wherein instead of step (e) the processing unit of the system is configured to perform the following step:
e'. compare the last measured lipid profile values of the patient with the personalized ranges of target lipid profile values of step (b), wherein if said last measured lipid profile values of the patient lies outside the personalized ranges of target lipid profile values, then provide a list of medication treatments wherein said list is sorted based on the evolution of the measured lipid profiles values with time, the effect of each medication treatment on the lipid profile of the patient, the difference between the measured lipid profile values and the personalized ranges of target lipid profile values, at least part of the clinical data of step (a), and the pharmacogenetic analysis of step (c).

9. The clinical decision support system according to any one of the previous claims, wherein the physiological data comprises data of the patient related to their sex and/or age.

10. The clinical decision support system according to any of the previous claims, wherein the prescribed medication comprises dyslipidaemia medication, hypolipidemic medication and/or anti-hypolipidemic medication.

11. The clinical decision support system according to any one of the previous claims, wherein it is received at least medical records and medication currently prescribed to the patient as clinical data, and wherein if the medical records comprise certain current medical conditions and the medication currently prescribed to the patient comprises lipid-lowering medication, then the processing unit of the system is further configured to output a safety warning indicating possible secondary effects of the medication, preferably wherein the medical conditions comprise at least one of myalgia, rhabdomyolysis and/or high transaminases.

12. The clinical decision support system according to any one of the previous claims, wherein the personalized ranges of target lipid profile comprises at least one first threshold subrange, wherein if at least one value of the measured lipid profile values of the patient is inside said at least one threshold subrange, and the patient does not have currently any dyslipidaemia medication prescribed, then do not provide a list of medication treatments and instead output a recommendation of healthy lifestyle.

13. The clinical decision support system according to any one of the previous claims, wherein the personalized ranges of target lipid profile comprises at least one second threshold subrange, wherein if at least one value of the measured lipid profile values of the patient is inside said at least one threshold subrange, and the patient currently has one or more dyslipidaemia medication prescribed, then output a recommendation of healthy lifestyle and provide a list of medication treatments, preferably sorted based on the effect of each medication treatment on the lipid profile values of the patient, the threshold subrange in which the measured lipid profile lies, at least part of the clinical data of step (a), and/or the pharmacogenetic analysis of step (c).

14. Computer program comprising instructions which, when executed by a processor, causes the processor to carry out the steps comprised in the configuration of the processing unit according to any one of the previous claims.

15. A computer readable storage medium comprising instructions which, when executed by a processor, cause the computer to carry out the steps comprised in the configuration of the processing unit according to any one of the previous claims.
